# FASCICULE DE BREVET EUROPEEN

(11) **EP 2 173 887 B1**
(45) Date de publication et mention de la délivrance du brevet: **04.12.2013**
(21) Numéro de dépôt: 08786548.1
(22) Date de dépôt: 29.07.2008
(51) Int. Cl.: C12N 15/82, C12N 15/29, C12N 9/04, A23K 3/00, A01H 5/00, A01H 5/10

(54) **MAÏS PRESENTANT UNE DIGESTIBILITE AMELIOREE**
MAIS MIT VERBESSERTER VERDAULICHKEIT
MAIZE HAVING IMPROVED DIGESTIBILITY

(30) Priorité: 31.07.2007 FR 0705601
(43) Date de publication de la demande: 14.04.2010
(73) Titulaire: Biogemma, 75001 Paris (FR)
(72) Inventeur: MURIGNEUX, Alain, F-63670 La Roche Blanche (FR); MARTINANT, Jean-Pierre, F-63910 Vertaizon (FR); TATOUT, Christophe, F-42110 Salt en Donzy (FR)
(74) Mandataire: Flesselles, Bruno F.G.
(86) Numéro de dépôt international: PCT/EP2008/059909
(87) Numéro de publication internationale: WO 2009/016166

(56) Documents cités:
- WO-A-01/95702
- WO-A-03/008585
- HALPIN CLAIRE ET AL: "Brown-midrib maize (bm1) - a mutation affecting the cinnamyl alcohol dehydrogenase gene" PLANT JOURNAL, BLACKWELL SCIENTIFIC PUBLICATIONS, OXFORD, GB, vol. 14, no. 5, juin 1998 (1998-06), pages 545-553, XP002150097 ISSN: 0960-7412
- MCKIE J H ET AL: "A molecular model for cinnamyl alcohol dehydrogenase, a plant aromatic alcohol dehydrogenase involved in lignification." BIOCHIMICA ET BIOPHYSICA ACTA 3 SEP 1993, vol. 1202, no. 1, 3 septembre 1993 (1993-09-03), pages 61-69, XP002459439 ISSN: 0006-3002
- HALPIN C ET AL: "MANIPULATION OF LIGNIN QUALITY BY DOWNREGULATION OF CINNAMYL ALCOHOL DEHYDROGENASE" PLANT JOURNAL, BLACKWELL SCIENTIFIC PUBLICATIONS, OXFORD, GB, vol. 6, no. 3, 1994, pages 339-350, XP002054141 ISSN: 0960-7412
- LAUVERGEAT V ET AL: "Site-directed mutagenesis of a serine residue in cinnamyl alcohol dehydrogenase, a plant NADPH-dependent dehydrogenase, affects the specificity for the coenzyme." BIOCHEMISTRY 26 SEP 1995, vol. 34, no. 38, 26 septembre 1995 (1995-09-26), pages 12426-12434, XP002459440 ISSN: 0006-2960

## Description

La présente invention se rapporte au domaine de l'amélioration des plantes, en particulier de l'amélioration de la digestibilité du maïs. Etant donné l'importance qualitative de la lignine dans les propriétés de digestibilité, une augmentation, une diminution ou une modification de la quantité et/ou de la qualité de la lignine peut avoir des conséquences industrielles ou agronomiques importantes. La présente invention concerne plus précisément la mise au point d'un allèle particulier du gène codant pour la première isoforme de la Cinnamyl alcohol dehydrogenase ou CAD2 (EC : 1.1.1.195) chez le maïs. La présence de cet allèle a pour conséquence la diminution de la quantité de lignine présente dans la plante.

La lignine est l'un des deux composants majoritaires de la paroi végétale avec la cellulose. La paroi végétale principalement constituée de cellulose, hémicellulose et de lignine offre à la cellule une barrière naturelle contre l'extérieur. De nombreuses études ont démontré que l'une des réponses aux stress biotiques (attaques pathogènes) ou abiotiques (sécheresse, vent...) consiste en un renforcement de la paroi végétale en particulier en une teneur supérieure en lignines. Par ailleurs, de nombreux secteurs agronomiques ou industriels voient leurs rendements directement liés à la teneur et/ou à la composition en lignine de la paroi. Parmi eux, il est possible de citer les industries papetières, la production de combustibles ou la production d'ensilage.

Par conséquent, il est intéressant de pouvoir moduler la teneur et la composition en lignines soit afin de renforcer les parois végétales pour améliorer la résistance aux stress soit au contraire d'affaiblir la paroi végétale afin de faciliter l'extraction de la cellulose ou d'autres composés chimiques (industrie papetière, production d'énergie) ou la digestibilité des fourrages (Baucher et al., 1998 Plant Mol Biol 39, 437-447)

Par exemple, on peut améliorer la qualité du maïs d'ensilage en diminuant la teneur en lignine ou en en modifiant la composition. L'ensilage de maïs est un aliment intéressant : le rendement au champ est relativement élevé, la récolte et le stockage sont aisés, les qualités nutritionnelles sont stables et peuvent aisément être complémentées en protéines par d'autres ensilages de fourrage ou par des tourteaux de soja. Une expérimentation menée par Emile (1995, Annales de zootechnie) démontre qu'alimenter du bétail avec un maïs plus digestible permet d'augmenter le niveau de production de lait de plus d'un kilogramme par jour et la prise de poids de 22 kg par rapport à une alimentation avec un maïs moins digestible. Ainsi, plus la variété est digestible, plus on augmente la production laitière, en diminuant la la perte de chair. L'optimisation des qualités de l'ensilage de maïs consiste ainsi à augmenter l'énergie nette fournie par ce type d'alimentation en améliorant sa digestibilité et donc en diminuant la teneur en lignine.

Ainsi, la sélection ou l'obtention de plantes de maïs plus digestibles, notamment dont la voie de biosynthèse des lignines est modifiée est un des axes privilégiés d'amélioration du maïs. Il convient toutefois que les plantes sélectionnées possèdent de bons rendements et soient peu sensibles aux divers stress (mécanique, hydrique...)

Il est cependant difficile de savoir comment modifier la voie de biosynthèse des lignines et de prévoir quelles seront les conséquences des modifications. En effet, la voie de biosynthèse des lignines reste une voie complexe, faisant intervenir un grand nombre de réactions enzymatiques (Dixon et al., 2001, Phytochemistry, 57(7), 1069-1084), et dont les éventuels mécanismes de compensation sont encore imparfaitement élucidés.

La lignine est considérée comme étant un polymère insoluble de 3 monomères d'alcools ou monolignols : l'alcool p-coumarylique (sous-unités H), l'alcool coniférylique (sous-unités G) et l'alcool sinapylique (sous-unités S), issus de la voie des phénylpropanoïdes (Neish, 1968, Constitution and Biosynthesis of lignin, eds New York, Springer Verlag l-43). Chaque type de précurseur peut former une variété de liaisons avec d'autres et ainsi constituer la lignine. D'autres liaisons peuvent également s'établir avec d'autres composés pariétaux (polysaccharides et protéines) pour former un réseau complexe tridimensionnel.

Les principales étapes de fabrication de lignine sont l'hydroxylation, la O-méthylation des cycles aromatiques puis la conversion de la chaîne latérale carboxyl en une fonction alcool.

L'hypothèse actuelle de la voie de biosynthèse des monolignols considère que le réseau métabolique aboutissant à la formation des sous-unités S et G fait intervenir des réactions successives d'hydroxylations et de O-méthylations à différents niveaux de l'oxydation de la chaîne latérale. Les enzymes du réseau incluent :
- des O-méthyltransférases distinctes : la Caffeic acid 3-O Méthyltransférase (COMT) également appelée 5-hydroxyconiferyl aldéhyde O-méthyl transférase (AldOMT) et la Caffeoyl coenzyme A 3-O méthyltransférase (CCoAOMT)
- des hydroxycinnamate coenzyme A ligases (4CL)
- une ou des férulate 5-hydroxylase (F5H) à cytochrome P450
- et plusieurs isoformes de Cinnamoyl Co A réductase (CCR) et de Cinnamyl alcool dehydrogenase (CAD):

La propriétés de ces différentes enzymes ont fait l'objet de revues (Boudet et al, 1995 New Phytol. 129, 203-236; Dixon et al, 2001, précédemment cité; Whetten et al., 1998 Annu Rev. Plant Physiol Plant Mol Biol, 49, 585 -609, Li et al., 2000 J. Biol Chem, 275, 6537-6545).

Depuis plusieurs années, on a tenté de modifier les teneur et composition des plantes en lignines en surexprimant ou sous exprimant un ou plusieurs gènes de la voie de biosynthèse des lignines (Anterola et Lewis, 2002, Phytochemistry 61, 221-294). Notamment, les demandes de brevets (WO 9924561, EP0516958, WO9305160, WO9305159, WO9712982) exposent différentes stratégies imaginées. Cependant, la surexpression ou la sous expression d'une ou plusieurs enzymes ne donnent pas toujours des résultats constants et prévisibles.

Une autre stratégie consiste à utiliser, dans les schémas de sélection variétale, des mutants du gène ciblé. Par exemple, le mutant naturel « brown midrib » (présentant une coloration rouge brun des tissus lignifiés) bm3 de maïs possède une insertion dans le gène codant pour la COMT-AldOMT. Connu depuis longtemps, le mutant bm1 (Jorgensen, 1931, Journal of the American Society of Agronomy 23, 549-557 ; Kuc et Nelson, 1964, Archives of Biochemistry and Biophysics 105, 103-113) possède, quant à lui, une mutation colocalisant au locus de la CAD (Halpin et al, Plant J. 1998, 14(5), 545-53 ; Guillaumie et al, Planta. 2007, 226(1), 235-50). Ces maïs bm 1 et bm3 diffèrent des maïs « normaux » par une teneur réduite en lignine mais leur rendement en champ est moindre. Ils sont, en outre, plus sensibles à la verse et montrent un manque de croissance et de vigueur en début de végétation et un retard à la floraison. Tous ces défauts en empêchent l'exploitation (Barrière et Argillier, Agronomie, 13, 865-876 (1993), Anterola et Lewis, cité précédemment).

La Cinnamyl Alcool Dehydrogenase (CAD) intervient dans la voie de biosynthèse des lignines pour convertir les p-coumaraldéhyde, coniferaldéhyde et sinapaldéhyde en alcool p-coumaryl, alcool coniféryl et alcool sinapyl respectivement. On a isolé au moins deux isoformes de l'enzyme CAD dans différentes espèces (et jusqu'à 16 isoformes chez *Arabidopsis*). Le rôle exact de ces différentes isoformes reste discuté. Certains auteurs ont proposé que ces différentes isoformes aient des affinités différentes pour les substrats mentionnés ci-dessus. Une autre hypothèse envisage des isoformes de CAD différentes selon le type cellulaire ou le stade de développement. Mais aucune de ces hypothèses n'a été clairement démontrée.

Chez le maïs, Guillaumie et al (Planta. 2007; 226(1):235-50) reportent l'existence de 5 gènes s'annotant Cinnamyl alcohol dehydrogenase dont un gène de type 2, un gène de type 1, et 3 gènes annotés Cinnamyl alcohol dehydrogenase like), et un gène annoté Sinapyl alcohol dehydrogenase (qui peut avoir une activité enzymatique similaire). Les CAD2 de type 2 semblent plus particulièrement impliquées dans la voie de biosynthèse des lignines. Toutefois Guillaumie et al montrent que le gène CAD de type 1 serait également dérégulé dans le mutant bm1 (mutant dans le gène CAD type2).

Les demandes de brevet WO93005159 et WO98003535 concernent entre autres, des plantes transgéniques manipulées pour ce qui est du gène de la CAD de maïs. Ces demandes ne décrivent pas comment obtenir des plantes de maïs possédant un gène de CAD muté, présentant une digestibilité améliorée et des propriétés agronomiques acceptables.

L'homme du métier est ainsi incapable de prévoir si une mutation spécifique de la CAD2 autre que la mutation connue bm1 situé dans le locus de ce gène mènera à un maïs plus digestible et présentant un intérêt agronomique.

La présente invention a pour objet de fournir à l'homme du métier un maïs qui possède effectivement une digestibilité améliorée, par la mise au point d'un allèle favorable de la CAD2 (appelé Δ314), l'insertion d'un transposon ayant été effectuée après le nucléotide 740 du gène dans SEQ ID N° 1, représentant un allèle (ADNc) codant pour cette enzyme. La partie codante s'étend des nucléotides 73 à 1176 de cette séquence, et l'insertion est située dans le dernier exon du gène.

La séquence d'un autre ADNc (séquence Y13733 de GenBank) est représentée par SEQ ID N° 2, la partie codante s'étendant des nucléotides 128 à 1231 pour cette séquence. Il est clair que ces séquences ne sont données qu'à titre d'exemples, et que l'homme du métier est à même d'identifier lui-même les séquences génomique et/ou d'ARNm de la CAD2 pour différentes variétés de maïs.

Des graines possédant l'allèle Δ314 ont été déposées au NCIMB Limited, Ferguson Building, Craibstone Estate, Bucksbum, Aberdeen, Scotland, AB21 9YA, UK, le 26 juillet 2007, selon les dispositions du Traité de Budapest, sous le numéro NCIMB 41491.

L'invention fournit notamment une plante de maïs possédant ledit allèle Δ314.

Cette plante montre une perturbation de l'expression du gène CAD2 et/ou de l'enzyme codée par ce gène de telle sorte que la teneur en lignine est diminuée d'au moins 5 %, de préférence d'au moins 7 %, de façon plus préférée d'au moins 10 %, et la fraction digestible des parois est augmentée de 5 %, de préférence d'au moins 7 %, de façon plus préférée d'au moins 12 %, par rapport à des plantes presque isogéniques ne présentant pas cet allèle menant à une telle inhibition de l'activité du gène CAD2.

Ainsi, la présente invention se rapporte notamment à une plante de maïs présentant une augmentation de la digestibilité (mesurée par NIRS) d'au moins 5 %, de préférence d'au moins 10 %, de façon plus préférée d'au moins 15 %. L'invention se rapporte également à une plante de maïs qui présente une augmentation de la digestibilité (mesurée par la méthode DINAG) d'au moins 5 %, de préférence d'au moins 10 %, de façon plus préférée d'au moins 13 %.

De façon préférée, le maïs selon l'invention est un maïs « élite ». L'homme du métier connaît bien la définition d'un maïs élite. Par maïs élite, on entend un maïs destiné à générer des hybrides destinés à être commercialisés par croisement avec un autre maïs élite. Un maïs élite est défini comme tel en relation avec le territoire envisagé pour la commercialisation, ainsi que le(s) caractère(s) agronomique(s) recherché(s) pour la descendance hybride. Il s'agit notamment d'un maïs pouvant être inscrit dans un catalogue de référence.

Ainsi, selon que la descendance est destinée à l'alimentation humaine ou animale, on recherchera par exemple respectivement un rendement en grains ou un rendement à l'hectare et une bonne digestibilité, lorsque l'on évalue la nature « élite du maïs ».

Afin de déterminer le caractère élite d'un maïs, on compare des hybrides obtenus à partir de celui-ci aux hybrides commerciaux de référence (vendus pour le même objectif dans la même région), par des essais en champs, par relevé et mesures de caractères agronomiques appropriés à l'objectif recherché. Un maïs est défini comme élite si les résultats obtenus paramètres étudiés pour un hybride obtenu par croisement dudit maïs sont supérieurs à 90 % aux résultats relevés pour les mêmes paramètres des hybrides de référence. Dans le cadre de la présente invention, on étudie notamment le caractère de digestibilité (digestibilité mesurée par NIRS (near infrared spectroscopy), par exemple).

La spectroscopie par proche infrarouge (NIRS) est la mesure de la longueur d'onde et l'intensité d'absorption de la lumière proche infrarouge par un échantillon, dans les domaines 800 nm - 2.5 µm (12,500 - 4000 cm⁻¹) range. Cette spectroscopie est typiquement utilisée pour des mesures quantitatives de groupes fonctionnels organiques, en particulier O-H, N-H et C=O. Cette méthode est couramment utilisée dans l'analyse de la digestibilité d'échantillons.

Ainsi, un maïs élite est un maïs rassemblant le maximum de caractéristiques agronomiques nécessaires pour une pénétration économique du marché visé. Le marché du maïs étant aujourd'hui un marché d'hybrides, l'évaluation du caractère élite d'un maïs s'effectue également par l'aptitude dudit maïs à la combinaison/production d'hybrides.

Ainsi, la présente invention se rapporte préférentiellement à un maïs élite destiné à la commercialisation d'hybrides pour l'alimentation animale et l'ensilage, présentant l'allèle Δ314. Ce maïs élite est donc homozygote pour l'allèle Δ314.

Dans un autre mode de réalisation, l'invention se rapporte à un maïs hybride obtenu par croisement de deux lignées parentes homozygotes, ledit maïs hybride présentant un allèle Δ314. Ce maïs hybride peut être homozygote (si chaque parent homozygote présente l'allèle Δ314) ou hétérozygote pour l'allèle Δ314.

La présente invention fournit également à l'homme de métier les moyens permettant de sélectionner les plantes de maïs possédant cette caractéristique de digestibilité améliorée. Il suffit en effet d'effectuer une PCR, ou un Southem Blot (hybridation de l'ADN génomique sur membrane) pour suivre la présence de l'insertion dans le dernier exon du gène codant pour la CAD2. L'homme du métier peut aisément déterminer les amorces et sondes permettant d'identifier la présence de l'allèle Δ314. L'invention se rapporte ainsi également à une méthode de suivi de l'allèle Δ314, par des techniques de biologie moléculaires, et notamment *via* la PCR en utilisant les amorces mentionnées dans les exemples.

L'invention fait également l'objet d'un procédé d'obtention de plantes de maïs possédant une digestibilité améliorée grâce à l'allèle Δ314.

L'invention permet de mettre en oeuvre une méthode pour obtenir une lignée de maïs présentant une digestibilité accrue, comprenant l'étape d'introgresser l'allèle Δ314, dans une lignée de référence, présentant un caractère agronomique de qualité. L'introgression du caractère est notamment réalisée par sélection, selon les méthodes connues dans l'art (croisements et auto-fécondation). On sélectionne notamment les plantes à l'aide de marqueurs moléculaires.

Le principe en est rappelé ci-dessous :
On réalise une série de back-cross entre la lignée élite et la lignée portant l'allèle Δ314 (site unique sur le chromosome 5L).

Au cours des rétrocroisements, on peut sélectionner les individus porteurs de l'allèle Δ314, et ayant recombiné le plus petit fragment de la lignée donneuse autour de cet allèle. En effet, grâce aux marqueurs moléculaires, on sélectionne les individus ayant pour les marqueurs proches du gène, le génotype de la lignée élite.

De plus, il est également possible d'accélérer le retour vers le parent élite grâce aux marqueurs moléculaires répartis sur l'ensemble du génome. A chaque rétrocroisement, seront choisis les individus ayant le plus de fragments issus du parent élite récurrent.

Avec une bonne mise en oeuvre, dès la quatrième génération, on peut obtenir une lignée quasi isogénique de la lignée élite, c'est-à-dire identique à la lignée élite de départ, mais ayant intégré le locus portant l'allèle Δ314.

Ainsi, dans un mode de réalisation préféré, ladite méthode comprend les étapes consistant à :
a) croiser une première lignée de maïs présentant l'allèle Δ314 avec une seconde lignée de maïs ne présentant pas ledit allèle,
b) effectuer un génotypage de la descendance obtenue et sélectionner les descendants présentant l'allèle Δ314 ayant le meilleur génome ratio pour ce qui est de ladite seconde lignée,
c) effectuer un rétrocroisement desdits descendants avec ladite seconde lignée de maïs,
d) répéter si nécessaire les étapes b) et c) jusqu'à obtenir une lignée isogénique de ladite seconde lignée de maïs, présentant l'allèle Δ314,
e) de façon optionnelle, effectuer une auto-fécondation afin d'obtenir une plante homozygote pour l'allèle Δ314.

Le génotypage de l'étape b) est effectué préférentiellement en utilisant des marqueurs moléculaires (marqueurs microsatellites par exemple), permettant de définir la part de chacun des deux parents dans la descendance. On sélectionne également, dans la descendance, les maïs qui possèdent le caractère génétique approprié pour ce qui est de l'allèle Δ314, de façon classique, par les méthodes de biologie moléculaire (telles que PCR ou Southern Blot).

De manière surprenante, il a été montré que la répétition des rétrocroisements entre les lignées sélectionnées à l'étape b) et le second maïs permet d'obtenir l'apparition d'un phénotype bien plus marqué au sein dudit second maïs.

Ce résultat est tout à fait surprenant car on aurait pu s'attendre à observer une amélioration de la digestibilité dès le premier croisement du maïs présentant l'allèle Δ314 avec le second maïs.

Par ailleurs, les résultats agronomiques observés après de multiples rétrocroisements (5 rétrocroisements et 2 auto-fécondations) ne montrent pas de différence entre les lignées isogéniques présentant la mutation et les plantes témoins.

Ainsi, l'homme du métier ne pouvait penser pouvoir obtenir de maïs présentant une digestibilité accrue, avec des propriétés agronomiques satisfaisantes, à la vue de l'enseignement existant sur le mutant bm1. Il n'aurait pas non plus été incité à mettre au point un allèle spécifique mutant dans le gène CAD2, tel que celui décrit dans la présente invention.

Enfin, l'invention se rapporte à l'utilisation d'un maïs selon l'invention, pour la préparation d'une composition destinée à l'alimentation du bétail, à une méthode pour la préparation d'une composition destinée à l'alimentation du bétail comprenant l'ensilage d'un maïs selon l'invention, ainsi qu'à la composition destinée à l'alimentation du bétail, ainsi obtenue.

### DESCRIPTION DES FIGURES

Figure 1 : illustration d'une méthode permettant le suivi de l'introgression de l'allèle Δ314. Résultats d'amplification.
Figure 2 : résultats NIR de l'introgression de l'allèle Δ314 sur la quantité de lignine.
Plantes mutantes : possédant l'allèle Δ314; plantes contrôles : plantes ne possédant pas cet allèle.
Figure 3 : résultats de l'introgression de l'allèle Δ314 sur la quantité de lignine (ADL, figure 3.A), et la digestibilité des parois (DINAG, figure 3.B). mutants : plantes possédant l'allèle Δ314; contrôles : plantes ne possédant pas cet allèle.

### EXEMPLES

### Description d'un maïs présentant une altération dans le gène de CAD2

Une lignée de maïs présentant une insertion de l'élément mutator à la position 740 de la séquence de référence SEQ ID N°1 est isolée. L'allèle ainsi obtenu est appelé Δ314.

Afin de déterminer si l'insertion est sous forme homozygote ou hétérozygote, un couple d'amorces a été défini : amorce sens CAD2 15 de séquence SEQ ID N° 3 : AGCACTTTGGGCTGACGAAC, en amont de l'insertion, et une amorce antisens CCR 14 de séquence SEQ ID N° 4 ACCATCCATCGTCTCATCTC, en aval de l'insertion.

En plus de ces deux amorces l'amorce OMuA SEQ ID N°5 : CTTCGTCCATAATGGCAATTATCTC spécifique de l'élément transposable endogène est utilisée. Cette amorce est dirigée vers « l'extérieur » du transposon.

Ces trois amorces peuvent être utilisées simultanément dans une expérience d'amplification PCR à partir d'ADN génomique (Température d'hybridation = 58°C). Le dépôt sur gel des produits d'amplification révèle :
- l'obtention d'une seule bande d'environ 800 pb de long pour des plantes dites sauvages à ce locus (c'est à dire n'ayant pas la mutation) ;
- l'obtention d'une bande d'environ 630 pb pour des plantes homozygotes mutantes, correspondant à l'amplification obtenue entre CAD2 15 et OmuA ;
- ou l'obtention des deux bandes pour des plantes hétérozygotes.

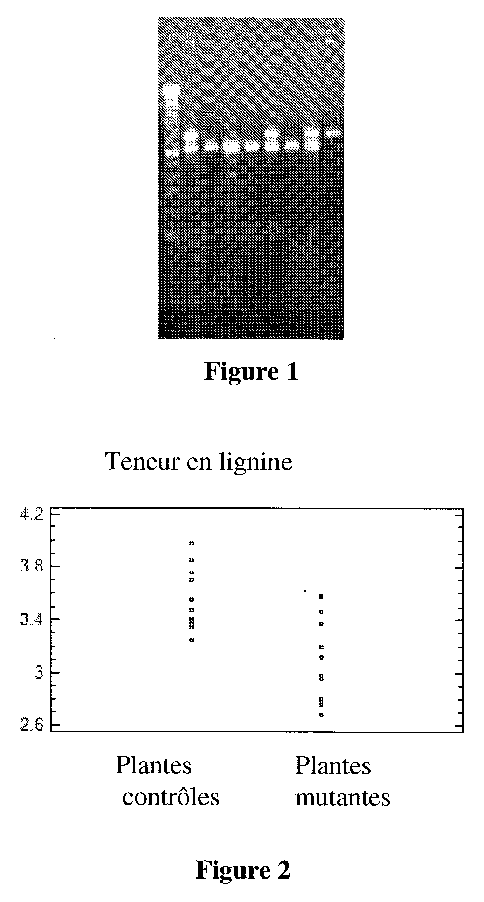

Ces résultats sont illustrés sur la Figure 1.
Le premier puits sur gel contient le marqueur de taille : la bande la plus basse correspond à 100 pb et il y a 100 pb entre chaque bande
Le puits 9 correspond à un individu sauvage ne portant pas l'allèle Δ314.
Les puits 3, 4, 5 et 7 correspondent à des individus homozygotes mutants pour Δ314.
Les puits 2, 6 et 8 correspondent à des individus hétérozygotes.

### Schéma des Back cross et autofécondations

Afin d'étudier plus précisément l'effet de l'insertion observée dans le gène CAD2 dans un maïs élite, on effectue des back-cross (rétrocroisements) successifs avec une lignée élite de Limagrain.

Cette méthode permet très rapidement d'obtenir des lignées quasi isogéniques ne différant que par le locus portant l'allèle modifié, les descendants étant testés pour posséder un ratio génome le plus proche possible de celui du parent élite tout en ayant l'allèle que l'on désire introgresser. Ces tests sont assistés par marqueurs moléculaires (techniques bien connues, microsatellites, AFLP...). Pour essayer d'apprécier l'effet de l'insertion au plus tôt (obtention de plantes homozygote), des autofécondations sont réalisées aux différents stades intermédiaires de back cross.

La figure 2 présente des résultats NIR obtenus sur des plantes BC2S2 (2 rétrocroisements et 2 auto-fécondations).

Ceci montre que l'introgression de l'allèle Δ314 permet d'obtenir une baisse de la quantité de lignine (Acid Détergent Lignin) après isolement des parois (Neutral Detergent Fibers), selon les méthodes connues de l'homme du métier (Figure 2). Différentes méthodes de prévision de digestibilité ont été notamment rappelées dans Andrieu et al (INRA Prod. Anim., 1999, 12 (5), 391-396).

La digestibilité des parois peut aussi être appréciée par la mesure du DINAG. Ce critère, proposé par Argillier et Barrière (DINAG, une estimation de la qualité de la partie non grain du maïs ensilage sur des échantillons de plante entière. Colloque Maïs Ensilage, Nantes, 17-18 septembre 1996), représente la digestibilité de la partie non amidon et non glucides solubles calculée de la manière suivante à partir de la solubilité enzymatique de la matière sèche de la plante entière :
DINAG % = 100 x (DCS % - Amidon % - Glucides sol. %) / (100 - Amidon % - Glucides sol. %) (la DCS est la solubilité enzymatique de la MS en %, mesurée par la méthode Aufrère (In vivo digestibility and prediction of digestibility of some by-products. Feeding value of by-products and their use by beef cattle. EEC Seminar. Gontrode, September 1983, 27-29.)).

Les résultats obtenus sur les plantes BC2S2 sont résumés dans le tableau suivant :

| | Plantes contrôles (moyenne) | Plantes mutantes (moyennes) |
|---|---|---|
| Teneur en lignine = ADL | 3.5 | 3.1 |
| Fraction digestible des parois | 39.8 | 44.7 |

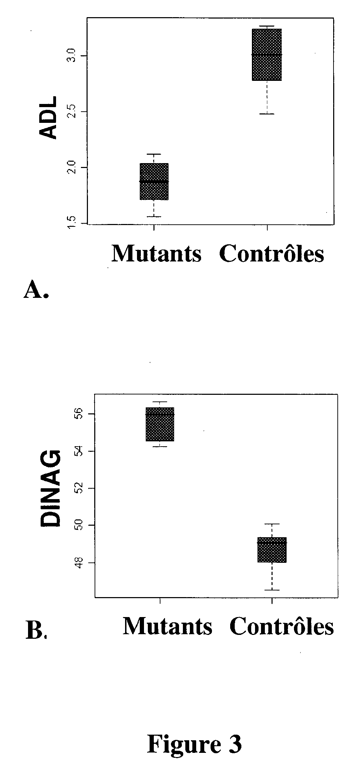

D'autres essais ont été réalisés sur des plantes BC6S2 (6 rétrocroisements et 2 auto-fécondations). Les résultats sont présentés sur la figure 3.

On peut ainsi noter que la diminution de la quantité de lignines est inversement corrélée à la digestibilité des paroi : moins de lignines se traduit par une meilleure digestibilité.

Ainsi, les résultats de la figure 3 montrent que l'introgression de l'allèle Δ314 permet d'obtenir non seulement une baisse du contenu en lignines (ADL, figure 3.A) mais aussi une amélioration de la digestibilité des parois (DINAG, figure 3.B). On peut observer une diminution de près de 35% du taux de lignines, et une amélioration de près de 14% du DINAG.

## Revendications

1. Maïs présentant un allèle du gène CAD2, appelé Δ314 comprenant une insertion d'un transposon après le nucléotide 740 de SEQ ID N°1, ledit allèle étant présent dans un échantillon représentatif de graines déposées au NCIMB sous le numéro NCIMB 41491.

2. Grain de maïs présentant un allèle du gène CAD2, appelé Δ314 comprenant une insertion d'un transposon dans le premier intron dudit gène, ledit allèle étant présent dans les graines déposées au NCIMB sous le numéro NCIMB 41491.

3. Utilisation d'un maïs selon la revendication 1 pour la préparation d'une composition destinée à l'alimentation du bétail.

4. Méthode pour la préparation d'une composition destinée à l'alimentation du bétail comprenant l'ensilage d'un maïs selon la revendication 1.

## Patentansprüche

1. Mais mit einem Allel des CAD2-Gens, wobei dieses Allel die Bezeichnung Δ314 tragt und umfassend eine Insertion eines Transposons nach Nukleotid 740 von SEQ ID N° 1, wobei das Allel in einer reprasentativen Samenprobe, die bei der NCIMB unter der Nummer NCIMB 41491 hinterlegt ist, vorhanden ist.

2. Maissamen mit einem Allel des CAD2-Gens, wobei dieses Allel die Bezeichnung Δ314 tragt und umfassend eine Insertion eines Transposons in dem ersten Intron des Gens, wobei das Allel in den Samen, die bei der NCIMB unter der Nummer NCIMB 41491 hinterlegt sind, vorhanden ist.

3. Verwendung eines Maises nach Anspruch 1 für die Herstellung einer Zusammensetzung, die für Haustierfutter bestimmt ist.

4. Verfahren zur Herstellung einer für Haustierfutter bestimmten Zusammensetzung, umfassend das Silieren eines Maises nach Anspruch 1.

## Claims

1. A maize exhibiting a CAD2 gene allele, called Δ314, comprising an insertion of a transposon after nucleotide 740 of SEQ ID N° 1, said allele being present in a representative sample of seeds deposited with NCIMB under number NCIMB 41491.

2. A seed of maize exhibiting a CAD2 gene allele, called Δ314, comprising an insertion of a transposon in the first intron of said gene, said allele being present in the seeds deposited with NCIMB under number NCIMB 41491.

3. Use of a maize according to Claim 1, for preparing a composition intended for livestock feed.

4. Method for preparing a composition intended for livestock feed, comprising the ensilage of a maize according to Claim 1.


### SEQUENCE LISTING

<110> BIOGEMMA
<120> Maïs présentant une digestibilité améliorée
<130> BGM 43 - WO
<150> FR 0705601
   <151> 2007-07-31
<160> 5
<170> PatentIn version 3.3
<210> 1
   <211> 1517
   <212> DNA
   <213> Zea mays
<220>
   <221> insertion
   <222> (740)..(741)
<400> 1
   gtgcgggctc gtctccatcg cccgccaccc gctccgtcgt cgttcgtccc cgccgcgccg      60
   atcccgaatc gaatggggag cctggcgtcc gagaggaagg tggtcgggtg ggccgccagg     120
   gacgccaccg gacacctctc cccctactcc tacaccctca ggaacacagg ccctgaagat     180
   gtggtggtga aggtgctcta ctgcgggatc tgccacacgg acatccacca ggcaaagaac     240
   cacctcgggg cttcaaagta tcctatggtc cctgggcacg aggtggtcgg cgaggtggtg     300
   gaggtcgggc ccgaggtggc caagtacggc gtcggcgacg tggtaggcgt cggggtgatc     360
   gttgggtgct gccgcgagtg cagcccctgc aaggccaacg ttgagcagta ctgcaacaag     420
   aagatctggt catacaacga cgtctacact gatggacggc ccacgcaggg tggattcgcc     480
   tccaccatgg tcgtcgacca gaagtttgtg gtgaagatcc cggcaggtct ggctccggag     540
   caagcggcgc cgctgctgtg cgctggcgtg acggtgtaca gcccgctgaa gcactttggg     600
   ctgacgaccc cgggcctccg tggcggcatc ctgggcctcg gcggcgtggg ccacatgggc     660
   gtgaaggtag ccaaggccat gggccaccac gtgacggtga tcagctcgtc gtccaagaag     720
   cgcgcggagg caatggacca cctcggcgcg gacgcgtacc tagtgagctc ggacgccgcg     780
   gccatggggc cggccgccga ctcgctggac tacatcatcg acacggtgcc cgtgcaccac     840
   ccgctggagc cgtacctggc gctgctgaag ctggacggca agctcgtgct gctgggcgtc     900
   atcggcgagc ccctgagctt cgtgtcgccc atggtgatgc tggggcggaa ggccatcacg     960
   gggagcttca tcggcagcat cgacgagacc gctgaggtgc ttcagttctg cgtcgacaag    1020
   gggctcacct cccagatcga ggtggtcaag atggggtacg tgaacgaggc gctggagcgg    1080
   ctggagcgca acgacgtccg ctaccgcttc gtcgtcgacg tcgccggtag caacgtcgag   1140
   gcggaggcgg cggcggcgga tgcggccagc aactgatggc accgcgtcgt cgagtcgaac   1200
   cacgtctgtg cgccgcgtgc aacgttcgtt cgggtcgagt ctgcgtgcaa cgttctgctt   1260
   cctttactag ttgttgtctt tccgccttct tgccgttctg ttctgggctt tgagatgaga   1320
   cgatggatgg tcagttttta atgtcagact gaataactac gtatagtact gtagtattac   1380
   tcggagtacg ccagaatgtg gtgtggtgtc agtctcacca gcaatctgga tttgccaagt   1440
   gtttctattt tttcttcggt ttgcccgagt gtttgtgatt gttaagaact acgttattac   1500
   ggatcgtcaa aaaaaaa                                                  1517
<210> 2
   <211> 1516
   <212> DNA
   <213> Zea mays
<400> 2
   ccggcgctcg cgcggctttc tttcccaact ccgacgaagg ctagctacac caccttgtgc      60
   gggctcgtct ccatcgcccg ccacccgctc cgtcgtcgtc gtccccgccg cgccgatccc     120
   gaatcgaatg gggagcctgg cgtccgagag gaaggtggtc gggtgggccg ccagggacgc     180
   caccggacac ctctccccct actcctacac cctcaggaac acaggccctg aagatgtggt     240
   ggtgaaggtg ctctactgcg ggatctgcca cacggacatc caccaggcca agaaccacct     300
   cggggcttca aagtatccta tggtccctgg gcacgaggtg gtcggcgagg tggtggaggt     360
   cgggcccgaa gtggccaagt acggcgtcgg cgacgtggta ggcgtcgggg tgatcgttgg     420
   gtgctgccgc gagtgcagcc cctgcaaggc caacgttgag cagtactgca acaagaagat     480
   ctggtcatac aacgacgtct acactgatgg acggcccacg cagggtggat tcgcctccac     540
   catggtcgtc gaccagaagt ttgtggtgaa gatcccggcg ggtctggctc cggagcaagc     600
   ggcgccgctg ctgtgcgctg gcgtgacggt gtacagcccg ctgaagcact ttgggctgac     660
   gaacccgggc ctccgtggcg gcatcctggg cctcggcggc gtgggccaca tgggcgtgaa     720
   ggtagccaag gccatgggcc accacgtgac ggtgatcagc tcgtcgtcca agaagcgcgc     780
   ggaggcaatg gaccacctcg gcgcggacgc gtacctagtg agctcggacg ccgcggccat     840
   ggcggcggcc gccgactcgc tggactacat catcgacacg gtgcccgtgc accacccgct     900
   ggagccgtac ctggcgctgc tgaagctgga cggcaagctc gtgctgctgg gcgtcatcgg     960
   cgagcccctg agcttcgtgt cgcccatggt gatgctgggg cggaaggcca tcacggggag    1020
   cttcatcggc agcatcgacg agaccgctga ggtgcttcag ttctgcgtcg acaaggggct    1080
   cacctcccag atcgaggtgg tcaagatggg gtacgtgaac gaggcgctgg agcggctgga    1140
   gcgcaacgac gtccgctacc gcttcgtcgt cgacgtcgcc ggtagcaacg tcgaggcgga    1200
   ggcggcggcg gcggatgcgg ccagcaactg atggcaccgc gtcgtcgagt cgaaccacgt    1260
   ctgtgcgccg cgtgcaacgt tcgttcgggt cgagtctgcg tgcaacgttc tgcttccttt    1320
   actagttgtt gtctttccgc cttcttgccg ttctgttctg ggctttgaga tgagacgatg    1380
   gatggtcagt ttttaatgtc agactgaata actacgtata gtactgtagt attactcgga    1440
   gtacgccaga atgtggtgtg gtgtcagtct caccagcaat ctggatttgc caagtgtttc    1500
   tattttttaa aaaaaa                                                    1516
<210> 3
   <211> 20
   <212> DNA
   <213> Artificial
<220>
   <223> primer CAD2 15
<400> 3
   agcactttgg gctgacgaac 20
<210> 4
   <211> 20
   <212> DNA
   <213> Artificial
<220>
   <223> primer CAD2 10
<400> 4
   accatccatc gtctcatctc 20
<210> 5
   <211> 25
   <212> DNA
   <213> Artificial
<220>
   <223> primer OmuA
<400> 5
   cttcgtccat aatggcaatt atctc 25